# FASCICULE DE BREVET EUROPEEN

(11) **EP 1 586 296 B1**
(45) Date de publication et mention de la délivrance du brevet: **30.03.2016**
(21) Numéro de dépôt: 05300266.3
(22) Date de dépôt: 08.04.2005
(51) Int. Cl.: A61K 8/25, A61K 8/29, A61K 8/44, A61Q 1/00, A61Q 1/02, A61Q 1/06, A61Q 1/10, A61Q 3/02, A61Q 5/10, A61K 8/02, A61K 8/11, A61K 8/19

(54) **Composition destinée à être appliquée sur la peau, les lèvres et/ou les phanères.**
Kosmetische Zusammensetzung für die Haut, die Lippen und/oder die Fingernägel
Cosmetic composition for application to the skin, lips and/or nails.

(30) Priorité: 08.04.2004 FR 0450713
(43) Date de publication de la demande: 19.10.2005
(73) Titulaire: L'Oréal, 75008 Paris (FR)
(72) Inventeur: Dumousseaux, Christophe, Tokyo (JP)
(74) Mandataire: Tanty, François

(56) Documents cités:
- EP-A- 0 581 651
- EP-A- 0 587 908
- EP-A- 1 184 426
- EP-A- 1 424 372
- EP-A- 1 433 462
- FR-A- 2 594 130
- US-B1- 6 428 773

## Description

La présente invention concerne les compositions cosmétiques destinées à être appliquées sur la peau, y compris les muqueuses, notamment les lèvres, et les phanères, notamment les ongles, cils, sourcils et cheveux.

Les matières colorantes organiques sont couramment utilisées pour apporter de la couleur aux compositions cosmétiques. Cependant, les matières colorantes organiques conventionnelles ne permettent pas d'obtenir des compositions présentant un pouvoir couvrant satisfaisant. De plus, les teintes apportées par ces matières colorantes organiques varient fortement entre l'état sec et l'état en dispersion dans un solvant cosmétique.

Il existe un besoin pour disposer de compositions cosmétiques présentant une saturation C* satisfaisante et un pouvoir couvrant suffisamment élevé.

Il existe encore un besoin pour disposer de pigments présentant un faible changement de couleur selon le milieu environnant

La présente invention vise à répondre à tout ou partie des besoins précités.

Elle y parvient grâce à une composition présentant un pouvoir couvrant avantageusement compris entre environ 25 et environ 100, par exemple entre environ 25 et environ 90, voire entre 30 environ et 100 environ, par exemple entre 30 et environ 90 environ, cette composition étant destinée à être appliquée sur la peau, les lèvres et/ou les phanères et comportant des particules d'au moins un pigment composite, ces particules comportant :
- un noyau inorganique,
- au moins un enrobage au moins partiel d'au moins une matière colorante organique dans une quantité suffisante pour que la saturation C* de la composition soit de préférence comprise entre environ 25 et environ 100, voire entre environ 30 et environ 100.

Grâce à l'invention, on peut obtenir des compositions à la fois saturées et couvrantes.

La teneur en pigment composite dans la composition peut être comprise entre environ 0,1 % et environ 30 %, voire entre environ 0,1 % et environ 20 %, par exemple entre environ 0,1 % et environ 15 %, voire entre environ 0,3 % et 10 % environ, par exemple entre environ 1 % et environ 5 % en poids, par rapport au poids total de la composition.

En particulier, on peut choisir une teneur en pigment composite relativement importante pour obtenir le pouvoir couvrant désiré et notamment un pouvoir couvrant relativement élevé.

Une teinte appropriée peut être obtenue de diverses façons, par exemple par le mélange de pigments composites selon l'invention, ces pigments ayant des couleurs différentes et/ou par la présence de plusieurs matières colorantes organiques dans l'enrobage des noyaux du ou des pigments composites, ces matières colorantes organiques étant par exemple mélangées ou présentes dans des strates respectives de l'enrobage.

Par «un enrobage au moins partiel », on entend au sens de la présente invention, un enrobage de tout ou partie du noyau inorganique.

La composition selon l'invention peut comporter un milieu physiologiquement acceptable.

Par « milieu physiologiquement acceptable », on désigne un milieu non toxique et susceptible d'être appliqué sur la peau, les lèvres ou les phanères d'êtres humains notamment un milieu cosmétique. Le milieu physiologiquement acceptable sera adapté à la nature du support sur lequel droit être appliquée la composition, ainsi qu'à la forme sous laquelle la composition est destinée à être conditionnée, notamment solide ou fluide à température ambiante et sous pression atmosphérique.

Par « composition cosmétique », on désigne une composition telle que définie dans la Directive 93/35/CEE du Conseil du 14 juin 1993.

### MESURE DE LA SATURATION C*

Dans le cas d'une composition liquide ou pâteuse, un échantillon de la composition à étudier est introduit dans une coupelle métallique d'environ 1 cm de profondeur. On applique sur la composition une lame de quartz de 1 mm d'épaisseur, en prenant soin d'éviter les bulles d'air avant de faire la mesure.

Dans le cas d'une composition en poudre, l'échantillon de poudre est compacté à une pression de 10 MPa dans une coupelle métallique d'environ 1 cm de profondeur. On applique sur la composition une lame de quartz de 1 mm d'épaisseur avant de faire la mesure.

Dans le cas d'un stick, la composition est coulée dans un moule en quartz à fond plat d'environ 2 cm de profondeur.

Les coordonnées trichromatiques L*, a*, b* de la composition dans l'espace CIE L*a*b* sont mesurées à l'aide d'un spectrocolorimètre CM-508d de MINOLTA, sous illuminant D65, avec une composante spéculaire incluse et un système d'éclairage d/8.

La saturation C* de la composition est calculée à l'aide de la formule C*=[(a*)²+(b*)²]^{1/2}.

### MESURE DU POUVOIR COUVRANT

Dans le cas d'un stick, la formulation est préalablement malaxée de façon à obtenir une pâte visqueuse.

Dans le cas d'une poudre, 50 parts en poids de la poudre sont malaxées avec 50 parts en poids de diméthicone (DC 200 Fluid 5CST de DOW CORNING) de façon à obtenir une pâte visqueuse.

La formulation est ensuite étalée avec une épaisseur de 30 µm sur une carte de contraste Erichsen, type 24/5, présentant un fond noir et un fond blanc, et les coordonnées trichromatiques (X, Y, Z) sont mesurées à l'aide d'un colorimètre CR-300.

Des étalements similaires sont réalisés sur deux autres cartes de contraste et trois mesures sont effectuées sur chaque carte. La moyenne correspondant à ces neuf mesures est ensuite calculée.

Le pouvoir couvrant est égal à 100 x Yn/Yb où Yn est la valeur moyenne de Y sur fond noir et Yb est la valeur moyenne de Y sur fond blanc. Un pouvoir couvrant de 100 correspond à une formulation complètement opaque.

### PIGMENT COMPOSITE

### Structure

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La proportion massique du noyau peut excéder 50 % par rapport au poids total du pigment composite, par exemple aller de 50 % à 70 %, par exemple de 60 % à 70 %.

La composition peut comporter uniquement un ou plusieurs pigments composites tels que définis plus haut ou en variante comporter un ou plusieurs pigments composites autres ainsi que des pigments présentant une structure non composite, notamment des pigments minéraux, interférentiels, des laques ou des pigments organiques. La composition peut notamment être dépourvue de particules de TiO₂ non enrobées.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite est supérieure ou égale à 30, mesurée selon le protocole ci-dessous.

### Protocole de mesure de la saturation du pigment composite :

Les valeurs a* et b* dans l'espace CIE L*a*b* du pigment composite sont mesurées comme suit :

Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a^{*2} + b^{*2})^{1/2}.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 1 nm et environ 100 nm, voire entre environ 5 nm et environ 75 nm, par exemple entre environ 10 nm et environ 50 nm, notamment 20 ou 25 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut présenter un indice de réfraction supérieur ou égal à 2, voire supérieur ou égal à 2,1, par exemple supérieur ou égal à 2,2.

Le noyau inorganique comporte des oxydes de titane

Les oxydes de titane, notamment TiO₂, conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau inorganique peut être coloré, le cas échéant.

### Matière colorante organique

La matière colorante organique peut comporter par exemple au moins un pigment organique, par exemple au moins une laque organique.

La matière colorante organique peut être choisie par exemple parmi les composés particulaires insolubles dans le milieu physiologiquement acceptable de la composition.

La matière colorante organique peut comporter par exemple des pigments, par exemple des laques organiques ou autres matières colorantes organiques, qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association ».

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La teneur totale en matière colorante organique de la composition, provenant du pigment composite et d'autres pigments éventuels, peut être par exemple inférieure à 10%, par rapport au poids total de la composition.

La proportion de la matière colorante organique peut excéder 30 % par rapport au poids total du pigment composite, par exemple aller de 30 à 50 %, par exemple de 30 à 40 %.

### Liant

Le liant peut être de tout type dès lors qu'il permet à la matière colorante organique d'adhérer à la surface du noyau inorganique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
   - les polysiloxanes modifiés (2A) comprenant au moins un radical choisi parmi, notamment, les polyéthers, les polyesters et les composés époxy (ils seront appelés « polysiloxanes modifiés »),
   - les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ₐ Si X₄₋ₐ (I)

dans laquelle :
- R¹ représente C₆H₅-, (CH₃)₂ CH-CH₂- ou un radical de type C_{b} H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi: le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) : 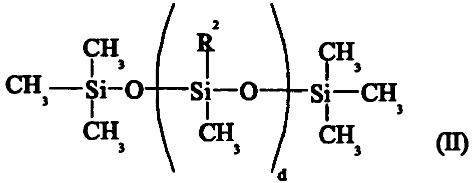 dans laquelle R² représente H- ou CH₃- et d varie de 15 à 450.

Parmi ces polysiloxanes, ceux pour lesquels R² représente H sont préférés.

Les polysiloxanes modifiés (2A) peuvent notamment répondre aux formules suivantes :
- (a¹) polysiloxanes modifiés portant des polyéthers, représentés par la formule (III) 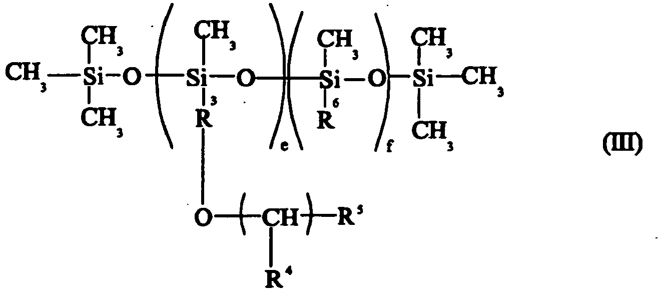 dans laquelle R³ représente -(CH₂)ₕ- ; R⁴ représente -(CH₂)ᵢ- CH₃ ; R⁵ représente -OH,-COOH, -CH = CH₂, -C (CH₃) = CH₂ ou -(CH₂)ⱼ- CH₃ ; R⁶ représente -(CH2)ₖ- CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 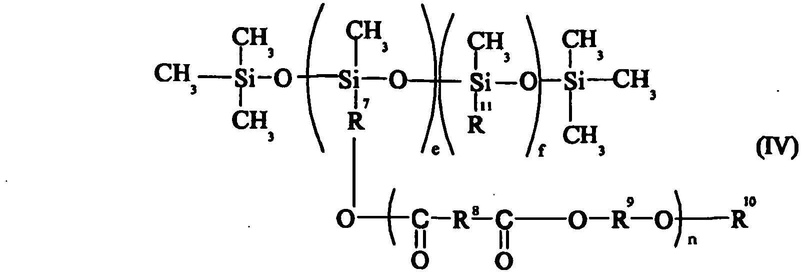 dans laquelle R⁷, R⁸ et R⁹ représentent indépendamment -(CH₂)_{q}- ; R¹⁰ représente -OH ; -COOH, -CH = CH₂, -C(CH₃) = CH₂ ou -(CH₂)ᵣ- CH₃ ; R¹¹ représente -(CH₂)ₛ- CH₃ ; n et q variant indépendamment de 1 à 15, r et s variant indépendamment de 0 à 15 ; e variant de 1 à 50 et f variant de 1 à 300,
- (a³) polysiloxanes modifiés portant des radicaux époxy représentés par la formule (V): 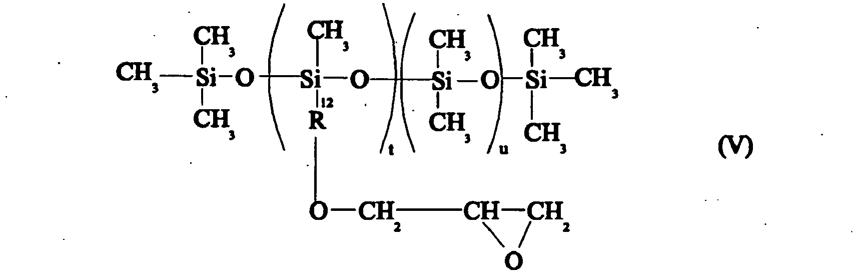 dans laquelle R¹² représente -(CH₂)ᵥ- ; v variant de 1 à 15 ; t variant de 1 à 50 et u variant de 1 à 300 ; ou leurs mélanges.
   Parmi les polysiloxanes modifiés (2A), les polysiloxanes modifiés portant des polyéthers de formule (III) sont préférés.
   Les polysiloxanes modifiés sur la partie terminale (2B) peuvent répondre à la formule (VI) : 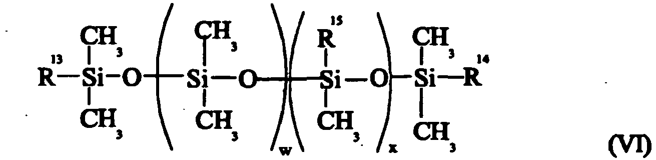 dans laquelle R¹³ et R¹⁴ peuvent représenter -OH, R¹⁶-OH ou R¹⁷-COOH, indépendamment l'un de l'autre ; R¹⁵ représente -CH₃ ou -C₆H₅ ; R¹⁶ et R¹⁷ représentent-(CH₂)_{y}- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂ (R¹⁸)ₐ SiX₄₋ₐ (VII)

dans laquelle : CF₃(CF₂)_{z}CH₂CH₂ (R¹⁸)ₐ SiX₄₋ₐ
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)-γ-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthyl-aminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant Pour de plus amples détails sur la manière de calculer la quantité relative du liant, on pourra se reporter à la demande EP 1 184 426 A2. La proportion relative de liant peut être inférieure ou égale à 5 %, par exemple inférieure ou égale à 3 %, par rapport au poids total du pigment composite.

### Préparation du pigment composite

Le pigment composite peut être préparé par tout procédé approprié, par exemple un procédé méchano-chimique ou un procédé de précipitation en solution, avec dissolution de la matière colorante organique puis précipitation à la surface du noyau.

Un liant peut être utilisé ou non.

Un procédé comportant un mélange mécanique d'un pigment organique et du noyau inorganique est préféré.

Un liant peut être ajouté et mélangé au noyau inorganique avant l'introduction de la matière colorante organique.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau inorganique avec le liant

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

### AUTRES COMPOSANTS

### Solvants

La composition peut comporter au moins un solvant aqueux ou organique.

Lorsque la composition comprend un ou plusieurs solvants organiques, ces solvants peuvent être présents en une teneur allant de 0,1 % à 99 %, par rapport au poids total de la composition.

D'une manière générale, la quantité de solvant(s), notamment organique(s), dépendra de la nature du support sur lequel la composition est destinée à être appliquée.

Dans le cas d'un vernis à ongles, par exemple, le solvant organique pourra être présent dans la composition à une teneur allant par exemple de 30 à 99 % en poids et de préférence de 60 % à 90 % en poids, par rapport au poids total de la composition.

La composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La composition peut, en outre, contenir des éthers en C₂ et des aldéhydes en C₂-C₄ hydrophiles. L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La composition, notamment lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes, et leurs mélanges. La phase grasse peut, en outre, contenir des solvants organiques lipophiles.

La composition peut présenter par exemple une phase grasse continue, pouvant contenir moins de 5% d'eau, notamment moins de 1% d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### Polymère filmogène

La composition peut encore comporter, par exemple, un polymère filmogène, notamment dans le cas d'un mascara ou d'un vernis à ongles. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans une composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

La composition selon l'invention peut comprendre également un agent auxiliaire de filmification favorisant la formation d'un film avec le polymère filmogène.

### Charges

La composition peut comprendre en outre des charges. Par « charges », on désigne des particules de toute forme, insolubles dans le milieu de la composition, quelle que soit la température à laquelle la composition est fabriquée. Ces charges peuvent servir notamment à modifier la rhéologie ou la texture de la composition. La nature et la quantité des corps solides sont fonction des propriétés mécaniques et des textures recherchées.

A titre d'exemple de charges, on peut citer, entre autres, le talc, le mica, la silice, le kaolin, la séricite, les poudres de polyamide, de polyéthylène, de polytétrafluoroéthylène, de polyméthacrylate de méthyle, de polyuréthane, les poudres d'amidon et les billes de résine de silicone.

### Matière colorante additionnelle

La composition peut comprendre une matière colorante additionnelle, différente du pigment composite utilisé dans la présente invention.

La matière colorante additionnelle peut être choisie parmi les pigments minéraux, les pigments organiques, les nacres, les colorants liposolubles ou hydrosolubles.

Les pigments minéraux peuvent être blancs ou colorés, enrobés ou on. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

Parmi les pigments organiques, on peut citer le noir de carbone, les pigments de type D & C, et les laques à base de carmin de cochenille, de baryum, strontium, calcium, aluminium.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent représenter de 0,1 à 20 % du poids de la composition et mieux de 0,1 à 6 % (si présents).

### Autres ingrédients

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, les colorants ou leurs mélanges.

La composition selon l'invention peut comprendre, selon le type d'application envisagée, les constituants classiquement utilisés dans les domaines considérés, qui sont présents en une quantité appropriée à la forme galénique souhaitée.

### Formes galéniques

La composition peut se présenter sous diverses formes, en fonction de sa destination. La composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau.

La composition peut se présenter sous forme de produit coulé, notamment de stick dans le cas d'un rouge à lèvres ou d'un produit de soin des lèvres.

La composition peut encore se présenter sous diverses autres formes, par exemple d'un liquide plus ou moins visqueux, d'un gel ou d'une pâte.

La composition peut encore se présenter sous la forme d'un solide, par exemple un pain à humidifier au moment de l'utilisation, de manière à lui permettre de se déliter.

La composition cosmétique peut constituer une composition de maquillage, entre autres, un rouge à lèvres, un gloss liquide, une pâte de rouge à lèvres, un fard à joues, un crayon à lèvres, un fond de teint solide ou fluide, un produit anti-cernes ou de contour des yeux, un eye-liner, un mascara, un vernis à ongles, une ombre à paupières, un produit de maquillage du corps ou des cheveux ou encore un produit solaire ou de coloration de la peau.

L'invention a ainsi encore pour objet un rouge à lèvres, liquide ou solide, comportant une composition telle que définie plus haut.

L'invention a encore pour objet un fond de teint comportant une composition telle que définie plus haut.

L'invention a encore pour objet un vernis à ongles comportant une composition telle que définie plus haut.

L'invention a encore pour objet un mascara comportant une composition telle que définie plus haut.

L'invention a encore pour objet un produit de coloration des fibres capillaires comportant une composition telle que définie plus haut.

L'invention a encore pour objet un procédé de maquillage de la peau, des lèvres ou des phanères, dans lequel on applique sur la peau, les lèvres ou les phanères une composition telle que définie plus haut

### EXEMPLES

On a réalisé, à titre illustratif, des compositions cosmétiques comportant des pigments composites avec les formulations suivantes, ces compositions étant préparées selon les procédés de préparation classiquement utilisés en cosmétique.

### Exemple 1 : Rouge à lèvres

Un rouge à lèvres ayant la composition suivante a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique (Softisan 100 de la société SASOL) | 5 |
| Octyldodécanol | 14,6 |
| Huile de lanoline | 8,9 |
| Huile de lanoline acétylée | 8,9 |
| Lanolate d'isopropyle | 8,9 |
| Trimellilate de tridécyle | 9,7 |
| Malate de diisostéaryle | 12,2 |
| Phényl triméthicone (DC 556 de DOW CORNING) | 4,0 |
| Pigment composite TiO₂/FD&C Blue 1 Al lake¹ | 15 |

| | |
|---|---|
| ¹Pigment composite constitué de 50 parts en poids de laque organique de dénomination FD&C Blue 1 Al lake pour 100 parts d'un noyau inorganique de dioxyde de titane de taille moyenne 20 nm et de surface spécifique pour 50 m²/g et réalisé avec un liant polyméthylhydrogénosiloxane. | |

### Exemple comparatif 1 : Rouge à lèvres

Un rouge à lèvres ayant la composition suivante, non conforme à l'invention car réalisée avec seulement pour matière colorante une laque organique pure, a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique (Softisan 100 de la société SASOL) | 5 |
| Octyldodécanol | 14,6 |
| Huile de lanoline | 8,9 |
| Huile de lanoline acétylée | 8,9 |
| Lanolate d'isopropyle | 8,9 |
| Trimellilate de tridécyle | 9,7 |
| Malate de diisostéaryle | 12,2 |
| Phényl triméthicone (DC 556 de DOW CORNING) | 4,0 |
| FD&C Blue 1 A1 lake² | 15 |

| | |
|---|---|
| ²Laque organique de dénomination FD&C Blue 1 A1 lake. | |

Les compositions de l'exemple 1 et de l'exemple comparatif 1 comportent les mêmes ingrédients dans les mêmes proportions, hormis les pigments.

La valeur de la saturation C* des compositions objets de l'exemple 1 et de l'exemple comparatif 1 a été mesurée. La différence de couleur ΔE par rapport à un pigment pur sous forme de poudre compactée a été également mesurée.

| | Exemple 1 | Exemple comparatif 1 |
|---|---|---|
| Saturation C* | 32,9 | 4,5 |
| ΔE/pigment pur | 8,9 | 36,2 |

La composition de l'exemple 1 présente une saturation C* élevée et une couleur peu différente de la couleur du pigment pur sous forme de poudre compactée, contrairement à la composition de l'exemple comparatif 1.

### Exemple 2 : Rouge à lèvres

Un rouge à lèvres ayant la composition suivante a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique (Softisan 100 de la société SASOL) | 5 |
| Octyldodécanol | 16,7 |
| Huile de lanoline | 10,3 |
| Huile de lanoline acétylée | 10,3 |
| Lanolate d'isopropyle | 10,3 |
| Trimellilate de tridécyle | 11,1 |
| Malate de diisostéaryle | 14,0 |
| Phényl triméthicone (DC 556 de la société DOW CORNING) | 4,5 |
| Pigment composite TiO₂/FD&C Blue 1 A1 lake ¹ | 5 |

| | |
|---|---|
| ¹Pigment composite constitué de 50 parts en poids de laque organique de dénomination FD&C Blue 1 A1 lake pour 100 parts d'un noyau inorganique de dioxyde de titane de taille moyenne 20 nm et de surface spécifique pour 50 m²/g et réalisé avec un liant polyméthylhydrogénosiloxane. | |

### Exemple comparatif 2 : Rouge à lèvres

Un rouge à lèvres ayant la composition suivante, non conforme à l'invention car réalisée avec seulement pour matière colorante une laque organique pure, a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL, & PITSCH) | 4 |
| Triglycéride d'acide palmitique-lamique-stéarique (Softisan 100 de la société SASOL) | 5 |
| Octyldodécanol | 16,7 |
| Huile de lanoline | 10,3 |
| Huile de lanoline acétylée | 10,3 |
| Lanolate d'isopropyle | 10,3 |
| Trimellilate de tridécyle | 11,1 |
| Malate de diisostéaryle | 14,0 |
| Phényl triméthicone (DC 556 de la société DOW CORNING) | 4,5 |
| FD&C Blue 1 A1 lake² | 5 |

| | |
|---|---|
| ²Laque organique de denomination FD&C Blue 1 A1 lake. | |

Les compositions de l'exemple 2 et de l'exemple comparatif 2 comportent les mêmes ingrédients dans les mêmes proportions, honnis les pigments.

On a mesuré le pouvoir couvrant et la saturation C* des compositions de l'exemple 2 et de l'exemple comparatif 2.

| | Exemple 2 | Exemple comparatif 2 |
|---|---|---|
| Pouvoir couvrant | 28 | 17 |
| Saturation C* | 31 | 8 |

Le pouvoir couvrant et la saturation C* ont des valeurs nettement plus élevées pour la composition de l'exemple 2 que pour celle de l'exemple comparatif 2.

### Exemple 3 : Fond de teint

Un fond de teint ayant la composition suivante a été préparé (quantités exprimées en % en poids par rapport au poids total de la composition) :

| | |
|---|---|
| Diméthicone copolyol (KF 6017 de ka société SHIN ETSU) | 5 |
| Diméthicone(DC 200 Fluid de la société DOW CORNING) | 4 |
| Cyclométhicone | 15 |
| Isododécane | 10 |
| Gel de bentone (Bentone gel VS5V de la société ELEMENTIS) | 10 |
| Plastic Powder D400³ | 4 |
| Oxyde de titane enrobé de stéaroyl glutamate d'aluminium⁴ | 6,61 |
| Oxyde de fer jaune enrobé de stéaroyl glutamate d'aluminium⁴ | 0,95 |
| Oxyde de fer noir enrobé de stéaroyl glutamate d'aluminium⁵ | 0,12 |
| Pigment composite TiO₂/D&C Red N°7⁵ | 0,32 |
| Eau | 33,2 |
| Butylène glycol | 10 |
| Sulfate de magnésium | 0,8 |

| | |
|---|---|
| ³commercialisé par la société TOSHIKI PIGMENT ⁴commercialisé par la société MIYOSHI ⁵pigment composite constitué de 50 parts en poids de pigment organique D&C Red N°7 pour 100 parts d'un noyau inorganique de dioxyde de titane de taille moyenne 20 nm et de surface spécifique 50 m²/g et réalisé avec un liant polyméthylhydrogénosiloxane. | |

### Exemple comparatif 3 : Fond de teint

Un fond de teint ayant la composition suivante, non conforme à l'invention car dépourvu de pigment composite, a été préparé (quantités exprimées en % en poids par rapport au poids total de la composition) :

| | |
|---|---|
| Diméthicone copolyol (KF 6017 de ka société SHUI ETSU) | 5 |
| Diméthicone(DC 200 Fluid de la société DOW CORNING) | 4 |
| Cyclométhicone | 15 |
| Isododécane | 10 |
| Gel de bentone (Bentone sel VS5V de la société ELEMENTIS) | 10 |
| Plastic Powder D400³ | 4 |
| Oxyde de titane enrobé de stéaroyl glutamate d'aluminium⁴ | 6,61 |
| Oxyde de fer jaune enrobé de stéaroyl glutamate d'aluminium⁴ | 0,95 |
| Oxyde de fer noir enrobé de stéaroyl glutamate d'aluminium⁴ | 0,12 |
| Oxyde de fer rouge enrobé de stéaroyl glutamate d'aluminium⁴ | 0,32 |
| Eau | 33,2 |
| Butylène glycol | 10 |
| Sulfate de magnésium | 0,8 |

| | |
|---|---|
| ³commercialisé par la société TOSHIKI PIGMENT ⁴commercialisé par la société MIYOSHI | |

Les compositions de l'exemple 3 et de l'exemple comparatif 3 comportent les mêmes ingrédients dans les mêmes proportions, hormis les pigments.

On a mesuré le pouvoir couvrant et la saturation C* des compositions de l'exemple 3 et de l'exemple comparatif 3.

| | Exemple 3 | Exemple comparatif 3 |
|---|---|---|
| Saturation C* | 27 | 22 |
| Pouvoir couvrant | 71 | 67 |

Le pouvoir couvrant et la saturation C* de la composition de l'exemple 3 sont supérieurs à ceux de la composition de l'exemple comparatif 3.

Bien entendu, l'invention n'est pas limitée aux exemples qui viennent d'être donnés.

Les intervalles donnés doivent être compris bornes incluses, sauf si le contraire est spécifié.

Dans toute la description, y compris les revendications, l'expression « comportant un » droit être comprise comme étant synonyme de « comportant au moins un », sauf si le contraire est spécifié.

## Revendications

1. Composition présentant un pouvoir couvrant compris entre 25 et 100, notamment entre 25 et 90, et destinée à être appliquée sur la peau, les lèvres et/ou les phanères, cette composition comportant des particules d'au moins un pigment composite, ces particules comportant :
- un noyau inorganique,
- au moins un enrobage au moins partiel d'au moins une matière colorante organique dans une quantité suffisante pour que la saturation C* de la composition (telle que définie dans la description) soit comprise entre 30 et 100 **caractérisé par le fait que**:
- le noyau inorganique présente une taille moyenne comprise entre 1 nm et 100 nm, un indice de réfraction supérieur ou égal à 2 et comporte du dioxyde de titane,
- la matière colorante organique est choisie parmi un carmin de cochenille, un pigment organique de colorant azoïque, anthraquinonique, indigoïde, xanthénique, pyrénique, quinolinique, de triphénylméthane, de fluorane, une laque organique ou un sel insoluble de sodium, de potassium, de calcium, de baryum, d'aluminium, de zirconium, de strontium, de titane, de colorant acide,
- la saturation C* du pigment (telle que définie dans la description) composite étant supérieure ou égale à 30,
- la proportion de la matière colorante organique excédant 30 % par rapport au poids total du pigment composite,
- le pouvoir couvrant étant mesuré de la manière suivante :
la composition est étalée avec une épaisseur de 30 µm sur une carte de contraste Erichsen, type 24/5, présentant un fond noir et un fond blanc, et les coordonnées trichromatiques (X, Y, Z) sont mesurées à l'aide d'un colorimètre CR-300 ;
des étalements similaires sont réalisés sur deux autres cartes de contraste et trois mesures sont effectuées sur chaque carte, la moyenne correspondant à ces neuf mesures est ensuite calculée, le pouvoir couvrant est égal à 100 x Yn/Yb où Yn est la valeur moyenne de Y sur fond noir et Yb est la valeur moyenne de Y sur fond blanc,
la teneur en pigment composite dans la composition étant comprise entre 0,1 % et 30 % en poids par rapport au poids total de la composition.

2. Composition selon la revendication 1, **caractérisée par le fait qu'**elle présente un pouvoir couvrant compris entre 30 et 100.

3. Composition selon l'une quelconque des revendications précédentes, **caractérisée par le fait que** la teneur en pigment composite dans la composition est comprise entre 0,1 % et 20 %, voire entre 0,1 % et 15 % en poids par rapport au poids total de la composition.

4. Composition selon l'une quelconque des revendications précédentes, **caractérisée par le fait que** la teneur en pigment composite dans la composition est comprise entre 0,3 % et 10 % en poids par rapport au poids total de la composition.

5. Composition selon l'une quelconque des revendications précédentes, **caractérisée par le fait que** la teneur en pigment composite dans la composition est comprise entre 1 % et 5 % en poids par rapport au poids total de la composition.

6. Composition selon l'une quelconque des revendications précédentes, **caractérisée par le fait que** la matière colorante organique comporte au moins un pigment organique.

7. Composition selon l'une quelconque des revendications précédentes, **caractérisée par le fait que** la matière colorante organique comporte au moins une laque organique.

8. Composition selon la revendication 1, **caractérisée par le fait que** le noyau inorganique présente une taille moyenne comprise entre 5 nm et 75 nm.

9. Composition selon la revendication8, **caractérisée par le fait que** le noyau inorganique présente une taille moyenne comprise entre 10 nm et 50 nm.

10. Composition selon la revendication 1, **caractérisée par le fait que** l'indice de réfraction du noyau inorganique est supérieur ou égal à 2,1.

11. Composition selon la revendication précédente, **caractérisée par le fait que** l'indice de réfraction du noyau inorganique est supérieur ou égal à 2,2.

12. Composition selon l'une quelconque des revendications précédentes, **caractérisée par le fait que** le noyau inorganique présente une surface spécifique comprise entre 1 m²/g et 1000 m²/g.

13. Composition selon la revendication précédente, **caractérisée par le fait que** la surface spécifique du noyau inorganique est comprise entre 10 m²/g et 600 m²/g.

14. Composition selon la revendication précédente, **caractérisée par le fait que** la surface spécifique du noyau inorganique est comprise entre 20 m²/g et 400 m²/g.

15. Composition selon l'une quelconque des revendications précédentes, **caractérisée par le fait que** le noyau inorganique comporte un matériau choisi parmi un sel métallique, un oxyde métallique, une alumine, un verre, une céramique, un graphite, une silice, un silicate, un mica synthétique, et un de leurs mélanges.

16. Composition selon la revendication précédente, **caractérisée par le fait que** le noyau inorganique comporte un oxyde métallique choisi parmi un oxyde de zirconium, de cérium, de zinc, de fer, de bleu ferrique, de chrome et d'aluminium.

17. Composition selon la revendication précédente, **caractérisée par le fait que** l'oxyde métallique est choisi parmi un oxyde de fer, de cérium, de zirconium, de zinc, et d'aluminium.

18. Composition selon la revendication 15, **caractérisée par le fait que** le noyau inorganique comporte au moins un silicate choisi parmi un aluminosilicate et un borosilicate.

19. Composition selon l'une quelconque des revendications précédentes, **caractérisée par le fait que** la proportion massique de la matière colorante organique est comprise entre environ 10 et environ 500 parts en poids pour 100 parts en poids du noyau inorganique.

20. Composition selon la revendication précédente, **caractérisée par le fait que** la proportion massique de la matière colorante organique est comprise entre environ 20 et environ 250 parts en poids pour 100 parts en poids du noyau inorganique.

21. Composition selon la revendication précédente, **caractérisée par le fait que** la proportion massique de la matière colorante organique est comprise entre environ 40 et environ 125 parts en poids pour 100 parts en poids du noyau inorganique.

22. Composition selon la revendication 15, **caractérisée par le fait que** le noyau inorganique comporte de la silice.

23. Composition selon l'une quelconque des revendications précédentes, **caractérisée par le fait que** le colorant acide est choisi parmi un colorant azoïque, anthraquinonique, indigoïde, xanthénique, pyrénique, quinolinique, de triphénylméthane, de fluorane, et tout autre colorant comportant au moins un groupe acide carboxylique ou sulfonique.

24. Composition selon l'une quelconque des revendications précédentes, **caractérisée par le fait que** la matière colorante organique comporte une laque organique qui est supportée par un support organique comportant au moins une colophane ou de benzoate d'aluminium.

25. Composition selon l'une quelconque des revendications précédentes, **caractérisée par le fait que** la matière colorante organique comporte un pigment organique ayant l'une des dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

26. Composition selon l'une quelconque des revendications précédentes, **caractérisée par le fait que** la matière colorante organique comporte une laque organique ayant l'une des dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

27. Composition selon l'une quelconque des revendications précédentes, **caractérisée par le fait que** ledit au moins un pigment composite comporte au moins un liant contribuant à la fixation de la matière colorante organique sur le noyau inorganique.

28. Composition selon la revendication précédente, **caractérisée par le fait que** le liant est organique.

29. Composition selon la revendication27 ou 28 , **caractérisée par le fait que** le liant comporte au moins l'un d'un composé siliconé, d'un composé polymérique, d'un composé oligomérique et similaire comportant au moins l'un d'un organosilane, d'un organosilane fluoroalkylé, d'un polysiloxane, d'un agent couplant, ou d'un mélange de ceux-ci.

30. Composition selon la revendication précédente, **caractérisée par le fait que** l'agent couplant est à base de silane, de titanate, d'aluminate, et/ou de zirconate, ou d'un mélange de ceux-ci.

31. Composition selon la revendication 29, **caractérisée par le fait que** le liant comporte au moins un composé siliconé.

32. Composition selon la revendication 29, **caractérisée par le fait que** le liant comporte du polyméthylhydrogénosiloxane.

33. Composition selon l'une quelconque des revendications précédentes, **caractérisée par le fait qu'**elle est dépourvue de particules de dioxyde de titane non enrobées.

34. Composition selon l'une quelconque des revendications précédentes, **caractérisée par le fait qu'**elle se présente sous une forme solide.

35. Composition selon l'une quelconque des revendications précédentes, **caractérisée par le fait qu'**elle se présente sous forme liquide, pâteuse ou gélifiée.

36. Composition selon l'une quelconque des revendications précédentes, **caractérisée par le fait que** le pigment composite est différent d'un pigment interférentiel.

37. Composition selon l'une quelconque des revendications précédentes, **caractérisée par le fait que** la teneur totale en matière colorante organique dans la composition est inférieure ou égale à 10 % en poids.

38. Composition telle que définie dans l'une quelconque des revendications précédente, **caractérisée en ce qu'**il s'agit d'un rouge à lèvres.

39. Composition telle que définie dans l'une quelconque des revendications 1 à 37 , **caractérisée en ce qu'**il s'agit d'un fond de teint.

40. Composition telle que définie dans l'une quelconque des revendications 1 à 37, **caractérisée en ce qu'**il agit d'un vernis à ongles.

41. Composition telle que définie dans l'une quelconque des revendications 1 à 37, **caractérisée en ce qu'**il s'agit d'un mascara.

42. Composition telle que définie dans l'une quelconque des revendications 1 à 37, **caractérisée en ce qu'**il s'agit d'un produit de coloration des fibres capillaires.

## Patentansprüche

1. Zusammensetzung, die ein Deckvermögen von 25 bis 100, insbesondere 25 bis 90 aufweist und zum Auftragen auf die Haut, die Lippen und/oder die Phaneren ausgelegt ist, die Teilchen von mindestens einem Verbundpigment umfasst, wobei diese Teilchen folgendes umfassen:
- einen anorganischen Kern,
- mindestens einen Überzug mindestens teilweise aus mindestens einem organischen Farbstoff in einer Menge, die ausreicht, dass die Sättigung C* der Zusammensetzung, wie in der Beschreibung definiert, von 30 bis 100 beträgt, **dadurch gekennzeichnet, dass**:
- der anorganische Kern eine durchschnittliche Größe von 1 nm bis 100 nm aufweist, einen Brechungsindex von größer oder gleich 2 aufweist und Titandioxid umfasst,
- der organische Farbstoff ausgewählt ist aus Karminlack, einem organischen Pigment von Azofarbstoff, Anthrachinon, Indigo, Xanthen, Pyren, Chinolin, Triphenylmethan, Fluoran, einem organischen Lack oder einem unlöslichen Salz von Natrium, Kalium, Calcium, Barium, Aluminium, Zirconium, Strontium, Titan, saurem Farbstoff,
- wobei die Sättigung C* des Verbundpigments, wie in der Beschreibung definiert, größer oder gleich 30 ist,
- der Anteil des organischen Farbstoffs, bezogen auf das Gesamtgewicht des Verbundpigments, über 30 % liegt,
- das Deckvermögen auf die folgende Weise gemessen wird:
die Zusammensetzung wird mit einer Dicke von 30 µm auf eine Erichsen-Kontrastkarte Typ 24/5 ausgestrichen, die einen schwarzen Hintergrund und einen weißen Hintergrund aufweist, und die trichromatischen Koordinaten (X, Y, Z) werden mit Hilfe eines CR-300-Kolorimeters gemessen;
gleiche Ausstreichungen werden auf zwei anderen Kontrastkarten durchgeführt und mit jeder Karte werden drei Messungen durchgeführt, der Mittelwert, der diesen neun Messungen entspricht, wird anschließend berechnet, das Deckvermögen beträgt 100 x Yn/Yb, wobei Yn der Mittelwert von Y auf schwarzem Hintergrund ist und Yb der Mittelwert von Y auf weißem Hintergrund ist,
wobei der Gehalt an Verbundpigment in der Zusammensetzung bezogen auf das Gesamtgewicht der Zusammensetzung 0,1 Gew.-% bis 30 Gew.-% beträgt.

2. Zusammensetzung nach Anspruch 1, **dadurch gekennzeichnet, dass** sie ein Deckvermögen von 30 bis 100 aufweist.

3. Zusammensetzung nach einem der vorangehenden Ansprüche, **dadurch gekennzeichnet, dass** der Gehalt an Verbundpigment in der Zusammensetzung 0,1 Gel.-% bis 20 Gew.-%, auch 0,1 Gew.-% bis 15 Gew.-% bezogen auf das Gesamtgewicht der Zusammensetzung beträgt.

4. Zusammensetzung nach einem der vorangehenden Ansprüche, **dadurch gekennzeichnet, dass** der Gehalt an Verbundpigment in der Zusammensetzung 0,3 Gel.-% bis 10 Gew.-% bezogen auf das Gesamtgewicht der Zusammensetzung beträgt.

5. Zusammensetzung nach einem der vorangehenden Ansprüche, **dadurch gekennzeichnet, dass** der Gehalt an Verbundpigment in der Zusammensetzung 1 Gel.-% bis 5 Gew.-% bezogen auf das Gesamtgewicht der Zusammensetzung beträgt.

6. Zusammensetzung nach einem der vorangehenden Ansprüche, **dadurch gekennzeichnet, dass** der organische Farbstoff mindestens ein organisches Pigment umfasst.

7. Zusammensetzung nach einem der vorangehenden Ansprüche, **dadurch gekennzeichnet, dass** der organische Farbstoff mindestens einen organischen Lack umfasst.

8. Zusammensetzung nach Anspruch 1, **dadurch gekennzeichnet, dass** der anorganische Kern eine durchschnittliche Größe von 5 nm bis 75 nm umfasst.

9. Zusammensetzung nach Anspruch 8, **dadurch gekennzeichnet, dass** der anorganische Kern eine durchschnittliche Größe von 10 nm bis 50 nm umfasst.

10. Zusammensetzung nach Anspruch 1, **dadurch gekennzeichnet, dass** der Brechungsindex des anorganischen Kerns größer oder gleich 2,1 ist.

11. Zusammensetzung nach einem der vorangehenden Ansprüche, **dadurch gekennzeichnet, dass** der Brechungsindex des anorganischen Kerns größer oder gleich 2,2 ist.

12. Zusammensetzung nach einem der vorangehenden Ansprüche, **dadurch gekennzeichnet, dass** der anorganische Kern eine spezifische Oberfläche von 1 m²/g bis 1000 m²/g aufweist.

13. Zusammensetzung nach einem der vorangehenden Ansprüche, **dadurch gekennzeichnet, dass** die spezifische Oberfläche des anorganischen Kerns 10 m²/g bis 600 m²/g beträgt.

14. Zusammensetzung nach einem der vorangehenden Ansprüche, **dadurch gekennzeichnet, dass** die spezifische Oberfläche des anorganischen Kerns 20 m²/g bis 400 m²/g beträgt.

15. Zusammensetzung nach einem der vorangehenden Ansprüche, **dadurch gekennzeichnet, dass** der anorganische Kern ein Material umfasst, das ausgewählt ist aus einem Metallsalz, einem Metalloxid, einem Aluminiumoxid, einem Glas, einer Keramik, einem Graphit, einem Siliciumoxid, einem Silicat, einem synthetischen Glimmer, und einem von ihren Gemischen.

16. Zusammensetzung nach einem der vorangehenden Ansprüche, **dadurch gekennzeichnet, dass** der anorganische Kern ein Metalloxid umfasst, das ausgewählt ist aus einem Oxid von Zirkonium, Cer, Zink, Eisen, Eisenblau, Chrom und Aluminium.

17. Zusammensetzung nach einem der vorangehenden Ansprüche, **dadurch gekennzeichnet, dass** der anorganische Kern ein Material umfasst, das ausgewählt ist aus einem Oxid von Eisen, Cer, Zirkonium, Zink, und Aluminium.

18. Zusammensetzung nach Anspruch 15, **dadurch gekennzeichnet, dass** der anorganische Kern mindestens ein Silicat umfasst, das ausgewählt ist aus einen Aluminosilicat und einem Borosilicat.

19. Zusammensetzung nach einem der vorangehenden Ansprüche, **dadurch gekennzeichnet, dass** der Masseanteil des organischen Farbstoffes etwas 10 bis etwa 500 Gewichtsteile auf 100 Gewichtsteile des anorganischen Kerns beträgt.

20. Zusammensetzung nach einem der vorangehenden Ansprüche, **dadurch gekennzeichnet, dass** der Masseanteil des organischen Farbstoffes etwas 20 bis etwa 250 Gewichtsteile auf 100 Gewichtsteile des anorganischen Kerns beträgt.

21. Zusammensetzung nach einem der vorangehenden Ansprüche, **dadurch gekennzeichnet, dass** der Masseanteil des organischen Farbstoffes etwas 40 bis etwa 125 Gewichtsteile auf 100 Gewichtsteile des anorganischen Kerns beträgt.

22. Zusammensetzung nach Anspruch 15, **dadurch gekennzeichnet, dass** der anorganische Kern Siliciumdioxid umfasst.

23. Zusammensetzung nach einem der vorangehenden Ansprüche, **dadurch gekennzeichnet, dass** der saure Farbstoff ausgewählt ist aus einem Azofarbstoff, Anthrachinonfarbstoff, Indigofarbstoff, Xanthenfarbstoff, Pyrenfarbstoff, Chinolinfarbstoff, Triphenylmethanfarbstoff, Fluoranfarbstoff, und jedem anderen Farbstoff, der mindestens eine Carbonsäuregruppe oder Sulfonsäuregruppe umfasst.

24. Zusammensetzung nach einem der vorangehenden Ansprüche, **dadurch gekennzeichnet, dass** der organische Farbstoff einen organischen Lack umfasst, der der von einem organischen Träger getragen wird, der mindestens ein Kolophonium oder Aluminiumbenzoat umfasst.

25. Zusammensetzung nach einem der vorangehenden Ansprüche, **dadurch gekennzeichnet, dass** der organische Farbstoff ein organisches Pigment mit einer der folgenden Bezeichnungen umfasst: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6.

26. Zusammensetzung nach einem der vorangehenden Ansprüche, **dadurch gekennzeichnet, dass** der organische Farbstoff ein organisches Pigment mit einer der folgenden Bezeichnungen umfasst:
D&C Red No. 2 Aluminium lake, D&C Red No. 3 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9 Aluminium lake, D&C Red No. 9 Barium lake, D&C Red No. 9 Barium/Strontium lake, D&C Red No. 9 Zirconium lake, D&C Red No. 10 Sodium lake, D&C Red No. 19 Aluminium lake, D&C Red No. 19 Barium lake, D&C Red No. 19 Zirconium lake, D&C Red No. 21 Aluminium lake, D&C Red No. 21 Zirconium lake, D&C Red No. 22 Aluminium lake, D&C Red No. 27 Aluminium lake, D&C Red No. 27 Aluminium/Titanium/Zirconium lake, D&C Red No. 27 Barium lake, D&C Red No. 27 Calcium lake, D&C Red No. 27 Zirconium lake, D&C Red No. 28 Aluminium lake, D&C Red No. 30 lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Blue No. 1 Aluminium lake, D&C Green No. 3 Aluminium lake, D&C Orange No. 4 Aluminium lake, D&C Orange No. 5 Aluminium lake, D&C Orange No. 5 Zirconium lake, D&C Orange No. 10 Aluminium lake, D&C Orange No. 17 Barium lake, D&C Yellow No. 5 Aluminium lake, D&C Yellow No. 5 Zirconium lake, D&C Yellow No. 6 Aluminium lake, D&C Yellow No. 7 Zirconium lake, D&C Yellow No. 10 Aluminium lake, FD&C Blue No. 1 Aluminium lake, FD&C Red No. 4 Aluminium lake, FD&C Red No. 40 Aluminium lake, FD&C Yellow No. 5 Aluminium lake, FD&C Yellow No. 6 Aluminium lake.

27. Zusammensetzung nach einem der vorangehenden Ansprüche, **dadurch gekennzeichnet, dass** das mindestens eine Verbundpigment mindestens ein Bindemittel umfasst, das zur Fixierung des organischen Farbstoffs an dem anorganischen Kern beiträgt.

28. Zusammensetzung nach einem der vorangehenden Ansprüche, **dadurch gekennzeichnet, dass** das Bindemittel organisch ist.

29. Zusammensetzung nach Anspruch 27 oder 28, **dadurch gekennzeichnet, dass** das Bindemittel mindestens eines von einer siliconisierten Verbindung, einer Polymemerbindung, einer Oligomemerbindung, und ähnlichem umfassend mindestens eines von einem Organosilan, einem Fluoralkylorganosilan, einem Polysiloxan, einem Kupplungsmittel, oder einem Gemisch von diesen umfasst.

30. Zusammensetzung nach einem der vorangehenden Ansprüche, dadurch gekennzeich-net, dass das Kupplungsmittel auf Silanbasis, Titanatbasis, Aluminatbasis und/oder Zirkonatbasis oder auf der Basis eines Gemisches von diesen ist.

31. Zusammensetzung nach Anspruch 29, **dadurch gekennzeichnet, dass** das Bindemittel mindestens eine siliconisierte Verbindung umfasst.

32. Zusammensetzung nach Anspruch 29, **dadurch gekennzeichnet, dass** das Bindemittel mindestens Polymethylhydrogensiloxan umfasst.

33. Zusammensetzung nach einem der vorangehenden Ansprüche, **dadurch gekennzeichnet, dass** in ihr nichtüberzogenen Titandioxid-Teilchen fehlen.

34. Zusammensetzung nach einem der vorangehenden Ansprüche, **dadurch gekennzeichnet, dass** sie in einer festen Form vorliegt.

35. Zusammensetzung nach einem der vorangehenden Ansprüche, **dadurch gekennzeichnet, dass** sie in flüssiger, pastöser oder gelierter Form vorliegt.

36. Zusammensetzung nach einem der vorangehenden Ansprüche, **dadurch gekennzeichnet, dass** sich das Verbundpigment von einem Interferenzpigment unterscheidet.

37. Zusammensetzung nach einem der vorangehenden Ansprüche, **dadurch gekennzeichnet, dass** der Gesamtgehalt an organischem Farbstoff in der Zusammensetzung kleiner oder gleich 10 Gew.-% beträgt.

38. Zusammensetzung nach einem der vorangehenden Ansprüche, **dadurch gekennzeichnet, dass** es sich um einen Lippenstift handelt.

39. Zusammensetzung nach einem der Ansprüche 1 bis 37, **dadurch gekennzeichnet, dass** es sich um eine Teintgrundierung handelt.

40. Zusammensetzung nach einem der Ansprüche 1 bis 37, **dadurch gekennzeichnet, dass** es sich um einen Nagellack handelt.

41. Zusammensetzung nach einem der Ansprüche 1 bis 37, **dadurch gekennzeichnet, dass** es sich um einen Mascara handelt.

42. Zusammensetzung nach einem der Ansprüche 1 bis 37, **dadurch gekennzeichnet, dass** es sich um eine Färbeprodukt der Haarfasern handelt.

## Claims

1. A composition having covering power comprised between 25 and 100, notably between 25 and 90 and intended to be applied on the skin, the lips, the skin appendages, this composition including particles of at least one composite pigment, these particles including:
- an inorganic core,
- at least one at least partial coating of at least one organic coloring substance in a quantity sufficient for the saturation C* of the composition, as defined in the description, to be comprised between 30 and 100, **characterized by** the fact that:
- the inorganic core has a mean size comprised between 1 nm and 100 nm, a refractive index greater than or equal to 2 and includes titanium dioxide,
- the organic coloring substance is selected from carmine cochineal, an organic pigment of an azo, anthraquinone, indigo, xanthene, pyrene, quinoline, triphenylmethane or fluorane dye, an organic lake, or an insoluble sodium, potassium, calcium, barium, aluminum, zirconium, strontium or titanium salt, of an acid dye,
- the saturation C* of the composite pigment, as defined in the description, being greater than or equal to 30,
- the proportion of the organic coloring substance exceeding 30% based on the total weight of the composite pigment,
- the covering power being measured in the following way:
the composition is applied with a thickness of 30 µm over an Erichsen type 24/5 contrast card, having a black background and a white background, and the trichromatic coordinates (X, Y, Z) are measured by means of a colorimeter CR-300;
similar applications are made on two other contrast cards and three measurements are conducted on each card, the mean corresponding to these nine measurements is then calculated, the covering power is equal to 100 X Yn/Yb wherein Yn is the mean value of Y on a black background and Yb is the mean value of Y on a white background,
the composite pigment content in the composition being comprised between 0.1% and 30% by weight based on the total weight of the composition.

2. The composition according to claim 1, **characterized by** the fact that it has a covering power comprised between 30 and 100.

3. The composition according to any of the preceding claims, **characterized by** the fact that the composite pigment content in the composition is comprised between 0.1% and 20%, or even between 0.1% and 15% by weight based on total weight of the composition.

4. The composition according to any of the preceding claims, **characterized by** the fact that the composite pigment content in the composition is comprised between 0.3% and 10% by weight based on total weight of the composition.

5. The composition according to any of the preceding claims, **characterized by** the fact that the composite pigment content in the composition is comprised between 1% and 5% by weight based on total weight of the composition.

6. The composition according to any of the preceding claims, **characterized by** the fact that the organic coloring substance includes at least one organic pigment.

7. The composition according to any of the preceding claims, **characterized by** the fact that the organic coloring substance includes at least one organic lake.

8. The composition according to claim 1, **characterized by** the fact that the inorganic core has a mean size comprised between 5 nm and 75 nm.

9. The composition according to claim 8, **characterized by** the fact that the inorganic core has a mean size comprised between 10 nm and 50 nm.

10. The composition according to claim 1, **characterized by** the fact that the refractive index of the inorganic core is greater than or equal to 2.1.

11. The composition according to the preceding claim, **characterized by** the fact that the refractive index of the inorganic core is greater than or equal to 2.2.

12. The composition according to any of the preceding claims, **characterized by** the fact that the inorganic core has a specific surface area comprised between 1 m²/g and 1,000 m²/g.

13. The composition according to the preceding claim, **characterized by** the fact that the specific surface area of the inorganic core is comprised between 10 m²/g and 600 m²/g.

14. The composition according to the preceding claim, **characterized by** the fact that the specific surface area of the inorganic core is comprised between 20 m²/g and 400 m²/g.

15. The composition according to any of the preceding claims, **characterized by** the fact that the inorganic core includes a material selected from a metal salt, a metal oxide, an alumina, a glass, a ceramic, a graphite, a silica, a silicate, a synthetic mica, and a mixture thereof.

16. The composition according to the preceding claim, **characterized by** the fact that the inorganic core includes a metal oxide selected from an oxide of zirconium, cerium, zinc, iron, iron blue, chromium and aluminum.

17. The composition according to the preceding claim, **characterized by** the fact that the metal oxide is selected from iron, cerium, zirconium, zinc and aluminum oxide.

18. The composition according to claim 15, **characterized by** the fact that the inorganic core includes at least one silicate selected from an aluminosilicate and a borosilicate.

19. The composition according to any of the preceding claims, **characterized by** the fact that the mass proportion of the organic coloring substance is comprised between about 10 and about 500 parts by weight for 100 parts by weight of the inorganic core.

20. The composition according to the preceding claim, **characterized by** the fact that the mass proportion of the organic coloring substance is comprised between about 20 and about 250 parts by weight for 100 parts by weight of the inorganic core.

21. The composition according to the preceding claim, **characterized by** the fact that the mass proportion of the organic coloring substance is comprised between about 40 and about our hundred and 125 parts by weight for 100 parts by weight of the inorganic core.

22. The composition according to claim 15, **characterized by** the fact that the inorganic core includes silica.

23. The composition according to any of the preceding claims, **characterized by** the fact that the acid dye is selected from an azo, anthraquinone, indigo, xanthene, pyrene, quinoline, triphenylmethane or fluorane dye, and any other dye including at least one carboxylic or sulfonic acid group.

24. The composition according to any of the preceding claims, **characterized by** the fact that the organic coloring substance includes an organic lake which is supported by an organic support including at least one rosin or aluminum benzoate.

25. The composition according to any of the preceding claims, **characterized by** the fact that the organic coloring substance includes an organic pigment having one of the following denominations:: D&C Blue No.4, D&C Brown No.1, D&C Green No.5, D&C Green No.6, D&C Orange No.4, D&C Orange No.5, D&C Orange No.10, D&C Orange No.11, D&C Red No.6, D&C Red No.7, D&C Red No.17, D&C Red No.21, D&C Red No.22, D&C Red No.27, D&C Red No.28, D&C Red No.30, D&C Red No.31, D&C Red No.33, D&C Red No.34, D&C Red No.36, D&C Violet No.2, D&C Yellow No.7, D&C Yellow No.8, D&C Yellow No.10, D&C Yellow No.11, FD&C Blue No.1, FD&C Green No.3, FD&C Red No.40, FD&C Yellow No.5, and FD&C Yellow No.6.

26. The composition according to any of the preceding claims, **characterized by** the fact that the organic coloring substance includes an organic lake having one of the following denominations: D&C Red No.2 Aluminum lake, D&C Red No.3 Aluminum lake, D&C Red No.4 Aluminum lake, D&C Red No.6 Aluminum lake, D&C Red No.6 Barium lake, D&C Red No.6 Barium/Strontium lake, D&C Red No.6 Strontium lake, D&C Red No.6 Potassium lake, D&C Red No.7 Aluminum lake, D&C Red No.7 Barium lake, D&C Red No.7 Calcium lake, D&C Red No.7 Calcium/Strontium lake, D&C Red No.7 Zirconium lake, D&C Red No.8 Sodium lake, D&C Red No.9 Aluminum lake, D&C Red No.9 Barium lake, D&C Red No.9 Barium/Strontium lake, D&C Red No.9 Zirconium lake, D&C Red No.10 Sodium lake, D&C Red No.19 Aluminum lake, D&C Red No.19 Barium lake, D&C Red No.19 Zirconium lake, D&C Red No.21 Aluminum lake, D&C Red No.21 Zirconium lake, D&C Red No.22 Aluminum lake, D&C Red No.27 Aluminum lake, D&C Red No.27 Aluminum/Titanium/Zirconium lake, D&C Red No.27 Barium lake, D&C Red No.27 Calcium lake, D&C Red No.27 Zirconium lake, D&C Red No.28 Aluminum lake, D&C Red No.30 lake, D&C Red No.31 Calcium lake, D&C Red No.33 Aluminum lake, D&C Red No.34 Calcium lake, D&C Red No.36 lake, D&C Red No.40 Aluminum lake, D&C Blue No.1 Aluminum lake, D&C Green No.3 Aluminum lake, D&C Orange No.4 Aluminum lake, D&C Orange No.5 Aluminum lake, D&C Orange No.5 Zirconium lake, D&C Orange No.10 Aluminum lake, D&C Orange No.17 Barium lake, D&C Yellow No.5 Aluminum lake, D&C Yellow No.5 Zirconium lake, D&C Yellow No.6 Aluminum lake, D&C Yellow No.7 Zirconium lake, D&C Yellow No.10 Aluminum lake, FD&C Blue No.1 Aluminum lake, FD&C Red No.4 Aluminum lake, FD&C Red No.40 Aluminum lake, FD&C Yellow No.5 Aluminum lake, FD&C Yellow No.6 Aluminum lake.

27. The composition according to any of the preceding claims **characterized by** the fact that said at least one composite pigment includes at least one binder contributing to attaching the organic coloring substance onto the inorganic core.

28. The composition according to the preceding claim, **characterized by** the fact that the binder is organic.

29. The composition according to claim 27 or 28, **characterized by** the fact that the binder includes at least one from among a silicone compound, a polymeric compound, an oligomeric compound or the like including at least one from among an organosilane, a fluoro-alkylated organosilane, a polysiloxane or a coupling agent, or a mixture thereof.

30. The composition according to the preceding claim, **characterized by** the fact that the coupling agent is based on silane, titanate, aluminate, and/or zirconate, or a mixture thereof.

31. The composition according to claim 29, **characterized by** the fact that the binder includes at least one silicone compound.

32. The composition according to claim 29, **characterized by** the fact that the binder includes polymethylhydrogensiloxane.

33. The composition according to any of the preceding claims, **characterized by** the fact that it is free of any uncoated titanium dioxide particles.

34. The composition according to any of the preceding claims, **characterized by** the fact that it appears in solid form.

35. The composition according to any of the preceding claims, **characterized by** the fact that it appears in liquid, pasty or gelled form.

36. The composition according to any of the preceding claims, **characterized by** the fact that the composite pigment is different from an interferential pigment.

37. The composition according to any of the preceding claims, **characterized by** the fact that the total organic coloring substance content in the composition is less than or equal to 10% by weight.

38. The composition as defined in any one of the preceding claims, **characterized in that** this is a lipstick.

39. The composition as defined in any of claims 1 to 37, **characterized in that** this is a foundation.

40. The composition as defined in any of claims 1 to 37, **characterized in that** this is a nail polish.

41. The composition as defined in any of claims 1 to 37, **characterized in that** this is a mascara.

42. The composition as defined in any of claims 1 to 37, **characterized in that** this is a product for coloring capillary fibers.
